# EUROPEAN PATENT SPECIFICATION

(11) **EP 1 758 839 B1**
(45) Date of publication and mention of the grant of the patent: **01.07.2009**
(21) Application number: 04753039.9
(22) Date of filing: 24.05.2004
(51) Int. Cl.: C07C 2/66, C07C 15/107

(54) **ALKYLAROMATIC PROCESS WITH SOLID CATALYST REGENERATION AND WITH REMOVAL OF AROMATIC BYPRODUCTS**
ALKYLAROMATISCHES VERFAHREN MIT REGENERATION DES FESTEN KATALYSATORS UND ENTFERNEN VON AROMATISCHEN NEBENPRODUKTEN
PROCEDE D'ALKYLATION DE COMPOSES AROMATIQUES AVEC REGENERATION DE CATALYSEURS SOLIDES ET EXTRACTION DE SOUS-PRODUITS AROMATIQUES

(43) Date of publication of application: 07.03.2007
(73) Proprietor: UOP LLC, Des Plaines, IL 60017-5017 (US)
(72) Inventor: BOZZANO, Andrea G., Des Plaines, IL 60017-5017 (US); DETRICK, Kurt A., Des Plaines, IL 60017-5017 (US)
(74) Representative: Chung, Hsu Min
(86) International application number: PCT/US2004/016141
(87) International publication number: WO 2005/118514

(56) References cited:
- EP-A- 0 643 029
- US-A- 5 276 231
- US-A- 6 069 285
- US-B1- 6 417 420

## Description

### BACKGROUND

The invention relates to the alkylation of aromatic compounds with olefins using solid catalyst. About thirty years ago it became apparent that household laundry detergents made of heavily branched alkylbenzene sulfonates were gradually polluting rivers and lakes. Solution of the problem led to detergents made of linear alkylbenzene sulfonates (LABS) and later modified linear alkylbenzene sulfonates (MLABS), both of which were found to biodegrade more rapidly than the heavily branched variety. Today, detergents made using LABS and MLABS are known.

LABS are made from linear alkylbenzenes (LAB), and MLABS can be made from modified linear alkylbenzenes (MLAB). The petrochemical industry produces LAB by dehydrogenating linear paraffins to linear olefins and then alkylating benzene with the linear olefins in the presence of HF.

Over the last decade, environmental concerns over HF have increased, leading to a search for substitute processes employing catalysts other than HF that are equivalent or superior to the standard process. Solid alkylation catalysis to produce LAB, for example, is the subject of vigorous, ongoing research. Solid alkylation catalysts can also be used to produce MLAB and are also being researched vigorously. MLAB may be made by dehydrogenating slightly branched paraffins to slightly branched olefins and then alkylating benzene with the slightly branched olefins in the presence of a solid catalyst.

All alkylation catalysts, including HF and substitute catalysts for HF, lose some portion of their activity with continued use. The solid catalysts used to date in aromatic alkylation tend to deactivate rather quickly. Solid catalysts used for alkylation of aromatic compounds by olefins in the 6 to 22 carbon atom range usually are deactivated by gum-type materials that accumulate on the surface of the catalyst and block reaction sites. These materials include byproducts, such as aromatic (including polynuclear) hydrocarbons in the 10 to 22 carbon atom range, that are formed in the dehydrogenation of C6 to C22 paraffins. These materials also include undesired alkylation byproducts of higher molecular weight than the desired monoalkyl benzenes, e.g., di- and tri-alkyl benzenes, as well as olefin oligomers and other olefinic compounds.

An alkylation process using a solid alkylation catalyst typically includes means for periodically taking the catalyst out of service and regenerating it by removing these deactivating materials from the catalyst. For a solid alkylation catalyst, the catalyst life is measured in terms of time in service at constant conversion between regenerations. The longer the time between regenerations, the more desirable the catalyst and the process. It has been observed that the deactivating materials can be readily desorbed from the catalyst by washing the catalyst with the aromatic reactant (e.g., benzene). Catalyst reactivation or regeneration is effected by flushing the catalyst with benzene to remove the accumulated deactivating materials from the catalyst surface, generally with restoration of 100% of catalyst activity.

US-A-6069285 describes regenerating the solid catalyst in an aromatic alkylation process. The effluent of an alkylation reactor undergoing regeneration combines with the effluent of an on-stream alkylation reactor, and the combined effluent passes to a product recovery section of the process for recovering benzene, the alkylated benzene product, and other streams. The product recovery section comprises a benzene rectifier, a benzene fractionation column, and other product recovery facilities. Part of the benzene recovered from the product recovery section flows to the off-stream alkylation reactor to regenerate the deactivated catalyst. Another prior art process passes the effluent of the off-stream alkylation reactor to a separation zone to reject color bodies and to recover benzene that passes to the product recovery section. US-A-4072729 describes a continuous catalytic reaction and catalyst reactivation process using a simulated moving catalysts bed. Alkylation occurs in one zone of a multi-zone fixed catalyst bed and catalyst reactivation occurs simulteneously in another zone of the bed.

US-A-5276231 describes removing aromatic byproducts formed in the dehydrogenation of paraffins. These aromatic byproducts are believed to include, for example, alkylated benzenes, naphthalenes, other polynuclear aromatic, alkylated polynuclear hydrocarbons in the C10-C15 range, indanes, and tetralins, that is, they are aromatics of the same carbon number as the paraffin being dehydrogenated and may be viewed as aromatized normal paraffins. They are typically removed using an aromatics removal zone, comprising a fixed bed of sorbent.

US 6,417,420 describes a process for the production of alkylaromatic hydrocarbons by alkylating aromatic hydrocarbons with olefinic hydrocarbons. The olefinic hydrocarbons are produced by dehydrogenating paraffinic hydrocarbons. Aromatic byproducts formed in dehydrogenation are removed using an aromatic byproducts removal zone and either a dividing wall distillation column or thermally coupled distillation columns.

EP 0,643,029 describes a process of continuously alkylating in an alkylation step an aromatic compound in the presence of an alkylation catalyst with at least one linear olefin present in an alkylation feedstock. The alkylation feedstock is produced in a dehydrogenation step by dehydrogenation of at least one linear paraffin having from 6 up to 20 carbon atoms, and contains one or more unreacted linear paraffins and more than 2 weight percent accumulated aromatic byproducts formed in the dehydrogenation step.

Separating, and recycling benzene for the on-stream alkylation reactor catalyst regeneration is costly. The rewards of large reductions in capital investment and operating expenses are the incentive to developing new ways to use benzene more efficiently in aromatic alkylation processes.

### SUMMARY OF THE INVENTION

This invention is a solid catalyst alkylation process that makes multiple uses of feed aromatic that is used to regenerate the catalyst. In the first aspect there is provided a process for producing a alkyl aromatic, the process comprising:
a) producing a monoolefin and forming aromatic byproducts from an olefin-producing feed during the production of the monoolefin, and forming an olefinic stream comprising the monoolefin and aromatic byproducts;
b) removing aromatic byproducts from at least a portion of the olefin-producing feed or the olefinic stream in an on-stream aromatic byproducts removal bed containing a sorbent at sorption conditions effective to remove the aromatic byproducts;
c) passing a feed aromatic and at least a portion of the olefinic stream comprising the monoolefin to an on-stream alkylation reactor, alkylating the feed aromatic with the monoolefin in the presence of solid alkylation catalyst in the on-stream alkylation reactor at alkylation conditions to form a product aromatic, the alkylation conditions being sufficient to at least partially deactivate the solid alkylation catalyst in the on-stream alkylation reactor, and recovering from the on-stream alkylation reactor an on-stream reactor effluent stream comprising the product aromatic;
d) recovering the product aromatic from at least a portion of the on-stream reactor effluent stream;
e) contacting solid alkylation catalyst in an off-stream alkylation reactor with the feed aromatic, wherein the solid alkylation catalyst in the off-stream alkylation reactor is at least partially deactivated, wherein the off-stream alkylation reactor operates at regeneration conditions sufficient to at least partially reactivate the solid alkylation catalyst in the off-stream alkylation reactor, and recovering from the off-stream alkylation reactor an off-stream reactor effluent stream comprising the feed aromatic;
f) passing at least a portion of the off-scream reactor effluent stream to an off-stream aromatic byproducts removal bed containing sorbent, wherein the sorbent in the off-stream aromatic byproducts removal bed contains sorbed aromatic byproducts, to at least partially desorb the aromatic byproducts from the sorbent in the off-stream aromatic byproducts removal bed at desorption conditions, and recovering from the off-stream aromatic byproducts removal bed an off-stream bed effluent stream comprising the feed aromatic;
g) passing at least a portion of the feed aromatic in the off-stream bed effluent stream to the on-stream alkylation reactor;
h) at least intermittently shifting the functions of the on-stream alkylation reactor and the off-stream alkylation reactor by operating the on-stream alkylation reactor to function as the off-stream alkylation reactor and operating the off-stream alkylation reactor to function as the on-stream alkylation reactor; and
i) at least intermittently shifting the functions of the on-stream aromatic byproducts removal bed and the off-stream aromatic byproducts removal bed by operating the off-stream aromatic byproducts removal bed to function as the on-stream aromatic byproducts removal bed and operating the on-stream aromatic byproducts removal bed to function as the off-stream aromatic byproducts removal bed.

The subject invention can save capital investment and operating expenses compared to the prior art processes, since unnecessary separation, recovery and recycling of benzene are eliminated.

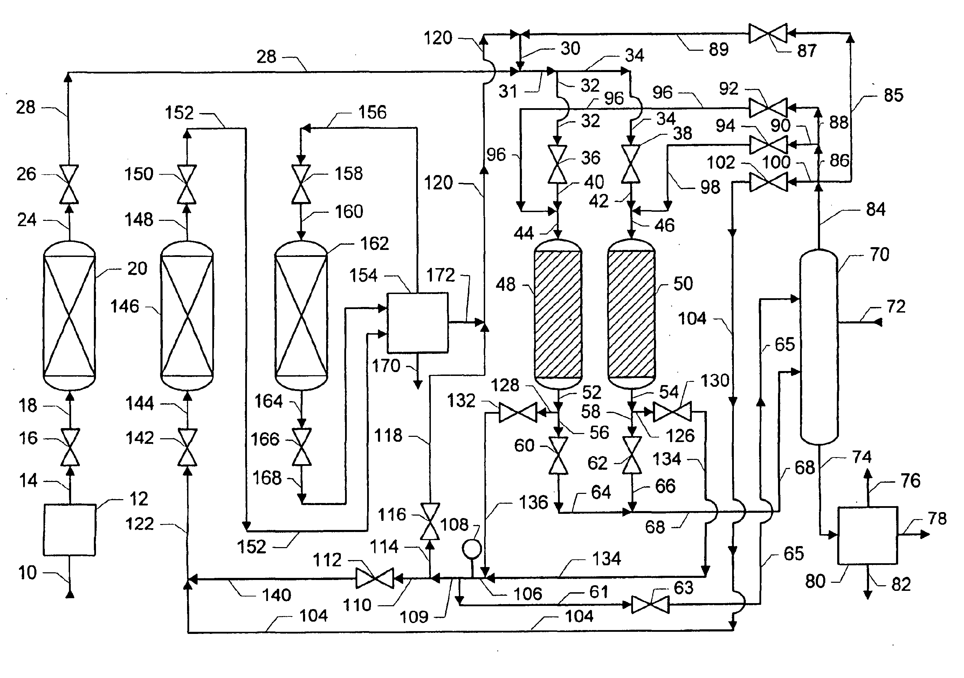

### BREF DESCRIPTION OF THE DRAWING

Ths drawing shows an embodiment of the invention.

### DETAILED DESCRIPTION OF THE INVENTION

US-A-6069285, US-B-6111158, and US-B-6187981 describe processes for the production of alkylated aromatic compounds, with and without the removal of aromatic byproducts. Briefly, an olefin feed stream reacts with an aromatic feed stream. The olefin feed stream contains C₆-C₂₂ monoolefins but may also contain C₆-C₂₂ paraffin and C₆-C₂₂ aromatic byproducts. The monoolefins may be linear or branched. When present, the branched monoolefins may have one or two methyl or ethyl branches, but more branches and branches with more carbon numbers are possible. The aromatic feed stream contains an aromatic compound, typically benzene or alkylated derivatives of benzene. The alkylated derivatives of benzene may include toluene, xylenes, and higher methylated benzenes, ethylbenzene and higher ethylated benzenes. In the discussion that follows and for purposes of illustration, the feed aromatic compound is referred to as benzene, because it is believed that this invention will be most commonly practiced using benzene.

Alkylation takes place in a selective alkylation reactor in the presence of the solid alkylation catalyst. The alkylation conditions may be any conditions suitable for the catalyst, but at least partial liquid phase conditions are preferred. The solid alkylation catalyst typically has an acid function and is better known as a solid acid catalyst. Suitable solid acid catalysts include amorphous silica-alumina, crystalline aluminosilicate materials such as zeolites and molecular sieves, naturally occurring and man-made clays including pillared clays, sulfated oxides such as sulfonated zirconia, traditional Friedel-Crafts catalysts such as aluminum chloride and zinc chloride, and solid acids generally. US-A-6069285 lists suitable solid alkylation catalysts. Such catalysts include those described in US-A-5196574 and US-A-5344997 disclose a fluorided silica-alumina catalyst, and US-A-5302732 describes an ultra-low sodium silica-alumina catalyst.

Two or more selective alkylation reactors are used, with at least one on-stream reactor where alkylation occurs and at least one off-stream reactor where catalyst regeneration takes place. Regeneration is effected by contacting the catalyst in the off-stream reactor with a stream comprising the feed aromatic compound (e.g., benzene).

After a reactor has been taken off-stream but before catalyst regeneration begins, the catalyst is typically purged to remove at least some of the unreacted monoolefins, paraffins, and alkylated benzene product from the void volume of the now off-stream reactor. Off-stream reactor purging is performed by contacting the catalyst in the off-stream reactor with any suitable hydrocarbon but preferably comprises the feed aromatic (e.g., benzene). The source of this purging stream can be any suitable supply of benzene, for example, such as a recycle stream from the hereinafter-described product recovery section, the effluent stream of another reactor (on-stream or off-stream), or an external supply of benzene.

Although olefins may contact the catalyst bed during off-stream reactor purging, preferably no olefins contact or pass to the catalyst during this purging. Preferably the off-stream reactor purging conditions comprise at least a partial liquid phase.

The contacting conditions for purging the catalyst in the off-stream reactor can change during the off-stream reactor purging. Although the liquid hourly space velocity (LHSV) of the benzene-containing stream may change, the contacting temperature is generally kept constant. This off-stream reactor purging can be started by simply stopping the flow of the olefinic feed stream to the on-stream reactor, thereby taking the on-stream reactor off-stream. This leaves substantially only benzene flowing to the off-stream reactor.

During off-stream reactor purging, the contacting temperature is preferably low enough that the previously mentioned gum-type materials that have accumulated on the catalyst are not removed. The off-stream reactor purging temperature is typically no more than 5°C (9°F) greater than the alkylation temperature. The temperature is usually between 120°C (248°F) and 170°C (338°F). In some portions of the alkylation reactor, the temperature during this step may be less than the temperature during alkylation. This is because there is less temperature rise due to less exothermic heat because less (or no) alkylation reactions are taking place.

During this off-stream reactor purging, the benzene-containing stream purges or displaces reactants and products from the void volume within the off-stream reactor. The composition of the off-stream reactor effluent will change during off-stream reactor purging. Initially, the effluent will consist mostly of benzene, unreacted monoolefin, paraffins, and alkylated benzene product. As the off-stream reactor purging proceeds and these components are displaced from the reactor, the reactor effluent will contain more benzene. As the reactor effluent contains more benzene, the Saybolt color of the reactor effluent may rise, indicating fewer color bodies in the reactor effluent. As used herein, color bodies are components of a mixture that impart color to the mixture, and Saybolt color refers to Saybolt color as determined by ASTM D-156-00, Standard Test Method for Saybolt Color of Petroleum Products (Saybolt Chronometer Method), which is available from ASTM International, 100 Barr Harbor Drive, P.O. Box C700, West Conshohocken, Pennsylvania, USA. This off-stream reactor purging may be deemed finished when the concentration of unreacted monoolefins, paraffins, and alkylated benzene product in the reactor effluent stream drops to a relatively low level as measured by gas chromatography, for example. Alternatively, the off-stream reactor purging may be considered done when at least one reactor void volume of benzene has flowed through the off-stream reactor during purging, when a specified period of time has elapsed, or when the Saybolt color has risen by a specified number or has reached a specified number.

During off-stream reactor purging, at least a portion of the effluent recovered from the off-stream reactor may pass to any suitable destination, such as to another reactor (on-stream or off-stream), to an external location, or to the section of the process that is normally used for separating the on-stream reactor effluent. This section is commonly called the product recovery section and it is used to recover benzene for recycling, unreacted monoolefins and paraffins for recycling, and the alkylated benzene as product. It typically comprises a benzene distillation column, a paraffin distillation column, and other distillation columns. The term portion as used herein in reference to a stream includes but is not limited to an aliquot portion of the stream, which is a portion of the stream that has the essentially the same composition as the stream.

Once off-stream reactor purging is complete, catalyst regeneration can begin. The regeneration conditions can be any conditions that are effective for at least partially reactivating the alkylation catalyst. Although olefins may contact the catalyst bed during regeneration, preferably no olefins contact or pass to the catalyst during regeneration. Preferably the regeneration conditions comprise at least a partial liquid phase.

The contacting conditions can be the same throughout the regeneration, but typically some changes in conditions are made. Commonly, the liquid hourly space velocity (LHSV) of the benzene-containing stream or the contacting temperature are changed during regeneration. Often, the LHSV of the benzene-containing stream is kept constant while the regeneration temperature is varied during the course of three steps.

The first step is a heat-up step. This step typically begins when the temperature of the off-stream reactor is increased above the temperature during off-stream reactor purging. Raising the temperature thus can mark the end of the off-stream reactor purging, if the purging has not already been deemed finished. A flow of the regenerant (i.e., the feed aromatic compound, such as benzene) is started to the off-stream reactor at or before the start of the heat-up step.

In this step, the regeneration temperature is higher than that during off-stream reactor purging but low relative to that during the second regeneration step. The temperature during this first step starts at the temperature at the end of the off-stream reactor purging. In this step, the inlet temperature is raised by from 50°C (90°F) to 200°C (360°F) above the temperature of the off-stream reactor purging. The outlet temperature of the bed increases with the inlet temperature but lags behind the inlet temperature. The inlet temperature is usually raised to a temperature that depends on the particular catalyst and the nature of the catalyst deactivation. For example, for a fluorided silica-alumina catalyst, the inlet temperature is usually raised to between 200°C (362°F) and the critical temperature of the aromatic feed compound (e.g., benzene), and preferably raised to 250°C (482°F). The inlet temperature may be increased at a rate that corresponds to raising the temperature from the off-stream reactor purging temperature to the target inlet temperature of the second regeneration step during the time period for passing a reactor void volume of benzene through the off-stream reactor. The temperature may be ramped up steadily or can be increased step-wise with temperature holds.

As the temperature increases, the benzene-containing stream begins to remove the previously mentioned gum-type materials that accumulated on the surface of the catalyst and block reaction sites. Since some of these gum-like materials typically have some color (i.e., are color bodies), the presence of these materials in the regeneration effluent may begin to lower its Saybolt color. This step may be deemed complete when the regeneration effluent's Saybolt color begins to decline. Alternatively, this step may be considered done when the inlet temperature has been increased by about a specified fraction (e.g., one-tenth, one-third, one-half, three-fourths) of the difference between the off-stream reactor purging temperature and the target inlet temperature of the second regeneration step. Since the composition of the regeneration effluent will change during this step, it is also possible to measure the concentration of the gum-like materials in the regeneration effluent stream, and to end this step when these materials have reach a specified concentration.

During this first step, at least a portion of the effluent recovered from the off-stream reactor passes to an on-stream reactor. In this way, substantially all of the components that are displaced from the off-stream reactor during this first step can be recovered in the effluent of the on-stream reactor. Of course, if the displaced benzene reacts in the on-stream reactor, what will be recovered in the on-stream reactor's effluent will include the product of any such reactions. In effect, this displaced benzene is used twice, for regenerating the catalyst in the off-stream reactor and for effecting alkylation within the on-stream reactor. Passing unreacted reactants such as benzene to the on-stream reactor gives them a second opportunity to react. But, regardless whether the displaced components react in the on-stream reactor or not, the fact that the on-stream reactor effluent passes to a benzene column and conventional facilities in the product recovery section means that the displaced components can be recovered and, if appropriate, recycled. Passing alkylated benzenes to the on-stream reactor does not adversely affect the yields of the process to a significant extent.

If a sufficiently large amount of benzene is passed to the off-stream reactor during the first step, then that benzene itself will appear in the off-stream reactor's effluent. That benzene in the effluent will be in addition to the benzene that was originally in the void volume of the off-stream reactor prior to the start of the first step. To the extent this happens, then at least a portion of the benzene passed to the off-stream reactor during the first step will have dual use. Not only is it used for regenerating the catalyst in the off-stream reactor, but it is used, or at least present, within the on-stream reactor effecting alkylation.

The second step continues the temperature heat-up and usually includes a hold period at the elevated temperature. In this step, the inlet temperature of the off-stream alkylation reactor is raised the rest (e.g., two-thirds, one-half, or one-fourth) of the way to the target inlet temperature of the second step, which is typically from 50°C (90°F) to 200°C (360°F) above the temperature of the first step. The target inlet temperature is also typically from 200°C (392°F) to the critical temperature of the feed aromatic compound (e.g., benzene). Preferably the rate of temperature increase during the heat-up portion of this step is substantially the same as that during the first regeneration step. Once the inlet temperature has reached its target inlet temperature, the temperature is typically held there for a specified period of time that depends on the nature of the catalyst and the extent and nature of the catalyst deactivation, typically from 2 to 20 hours. As the heat-up occurs, the outlet temperature of the reactor will lag behind the inlet temperature, but by the end of the hold period the outlet temperature will have generally stabilized and may have risen to a temperature close to that of the inlet temperature, depending on factors such as heat loss from the reactor. If the first regeneration step has not already been deemed finished by one of the previously-mentioned criteria, the time when the outlet temperature begins to increase in response to the raising of the inlet temperature (or a time preceding that time by some appropriate interval) can be used to mark the end of the first step.

As the temperature continues to increase and then is maintained at an elevated level, the benzene-containing stream continues to remove the gum-type materials from the surface of the catalyst. As the catalyst becomes depleted in these materials, the amount and concentration of these materials in the reactor effluent will decline. This second step can be said to be finished when the concentration of gum-like materials in the regeneration effluent stream drops to a relatively low level. Alternatively, this step can be considered complete when the time of the hold period has been reached when the regeneration effluent's Saybolt color, which had fallen at the start of the second step, begins to rise, or when a specified volume of benzene has flowed through the off-stream reactor during this step.

The third step is a cool-down step. The inlet regeneration temperature is reduced from the temperature at the end of the second step to the alkylation temperature. The temperature may be ramped down steadily or dropped step-wise with temperature holds. As the temperature decreases, less of the gum-type materials are removed from the catalyst. Typically, the temperature is decreased to a temperature that is suitable for re-introducing the olefinic feed stream to the reactor. When the olefinic feed stream is re-introduced, thereby putting the off-stream reactor back on-stream, this step is deemed completed.

This invention employs a sorptive aromatics removal zone to remove aromatic byproducts formed when producing monoolefins from an olefin-producing feed. Dehydrogenation of paraffins produces an olefinic stream. The olefin-producing feed can be fresh paraffins from an external source or a paraffin stream formed within the process, such as a paraffin recycle stream. During the second step and preferably also the third step, at least a portion of the effluent recovered from the off-stream reactor passes to an off-stream aromatics removal bed that is undergoing desorption. Aromatic byproducts are desorbed from the sorbent in the aromatics removal bed. Thus, any benzene that was passed to the off-stream reactor during the first, second, or third steps and which enters the off-stream aromatics removal bed for desorption without separation in the product recovery section has two uses. First it is used to purge or regenerate the catalyst in the off-stream reactor, and then it is used to desorb aromatic byproducts from the off-stream aromatics removal bed. These two uses occur without an intermediate separation of the benzene and in particular without separation in the product recovery section downstream of the alkylation reactors.

The desorption conditions can be any conditions that are effective for at least partially desorbing the aromatic byproducts from the sorbent. If the desorption temperature is different from the regeneration temperature, the effluent of the off-stream reactor can be heated or cooled as needed. Preferably the desorption conditions comprise at least a partial liquid phase. Suitable sorbents are disclosed in US-A-5276231, US-A-5334793, and US-A-6069285.

The effluent of the aromatics removal bed undergoing desorption typically passes to a separation section associated with the aromatics removal zone. This section usually comprises two distillation columns or a dividing wall distillation column. The aromatic byproducts in the desorption effluent are rejected from the process in a high-boiling stream. But the benzene, olefins, and paraffins that enter this separation section (via either the effluent of an aromatics removal bed undergoing desorption or the effluent of an aromatics removal bed undergoing purging) pass to an on-stream alkylation reactor to be used in the alkylation reaction. Thus, this invention allows for benzene that is passed to an off-stream reactor for regeneration to have as many as three uses before it passes to the product recovery section downstream of the alkylation reactors. The first use is for regenerating the catalyst in the off-stream reactor, the second use is for desorbing aromatic byproducts from an off-stream aromatics removal bed, and the third use is for effecting alkylation in an on-stream reactor.

When no alkylation reactor is undergoing regeneration, and particularly in the case of the previously described common three-step regeneration procedure when no alkylation reactor is undergoing either the second or third regeneration steps, there may nevertheless be a need for benzene to desorb an aromatics removal bed. Conversely, when no aromatics removal bed is being desorbed, an alkylation reactor may need to be regenerated. That is, there may be occasions when the times for desorption of an aromatics removal bed and for regeneration of an alkylation reactor do not coincide. In the former case, benzene for desorption may be taken from any suitable source, such as the overhead of the benzene column in the separation section downstream of the alkylation reactors. In the latter case, the regeneration effluent from the off-stream reactor may be passed (or may continue to be passed) to the on-stream alkylation reactor, may be passed to the previously mentioned benzene column, or may be passed to the separation section associated with the aromatic byproducts removal zone.

An aromatics byproducts removal zone may be placed in one or more locations in typical dehydrogenation-alkylation processes. Typical dehydrogenation-alkylation processes can use several optional zones or flow arrangements in which dehydrogenation forms some aromatic byproducts.

Where the dehydrogenated product stream passes to a stripping separation zone (e.g., a stripping column to remove light hydrocarbons), another location for the aromatic byproducts removal zone is on the stripping effluent stream that is recovered from the stripping separation zone. Third, where the overhead liquid stream of the paraffin column is recycled to the dehydrogenation zone, which is normally the case in commercial applications, the aromatic byproducts may be selectively removed from that recycle stream. Fourth, where the process includes a selective monoolefin hydrogenation zone, the aromatic byproducts may be selectively removed from the selective monoolefin hydrogenation product stream, which is recovered from that zone. Fifth, where the process includes a selective diolefin hydrogenation zone, the aromatic byproducts may be selectively removed from the selective diolefin hydrogenation product stream, which is recovered from that zone. The aromatics removal zone is preferably located between the dehydrogenation zone and the selective alkylation zone. But, regardless which location is selected for the bed of the aromatic byproducts removal zone in its sorption step, the bed's locations for its subsequent purging and desorption steps are as described herein.

At least a portion of the aromatic byproducts are removed so as to reduce the concentration of the aromatic byproducts in the olefinic feed stream to generally less than 2 wt-%, preferably less than 1 wt-%, and more preferably less than 0.5 wt-% aromatic byproducts.

The drawing shows two alkylation reactors 48 and 50. Each reactor contains solid alkylation catalyst. Reactor 48 is on-stream and in use for alkylating benzene with olefins. Reactor 50 is off-stream for off-stream reactor purging or regeneration of the catalyst. Valve 36 is open and valve 38 is closed, so that the olefin- and benzene-containing reactant stream flowing in line 31 passes through lines 32, 40, and 44 to on-stream reactor 48. Thus, there is no flow through lines 34 and 42 to off-stream reactor 50. Valve 60 is open and valve 132 is closed so that the effluent of on-stream reactor 48 passes through lines 52, 56, 64, and 68 to the benzene distillation column 70.

The drawing also shows three sorbent-containing beds, 20, 162, and 146. Each sorbent bed is performing a different function. Sorbent bed 20 is on-stream and functions to remove aromatic byproducts from a dehydrogenated product stream flowing in line 14.
Sorbent bed 162 is off-stream for off-stream sorbent bed purging, and a purging stream containing n-pentane, which flows in line 156, is purging its void volume. Sorbent bed 146 is also off-stream and aromatic byproducts on its sorbent are being desorbed by a desorbent stream containing benzene which flows in line 122. Each sorbent bed is shown with an inlet valve and an inlet line (16 and 18 for bed 20, 158 and 160 for bed 162, and 142 and 144 for bed 146, respectively), and an outlet line and an outlet valve (24 and 26 for bed 20, 164 and 166 for bed 162, and 148 and 150 for bed 146, respectively). The depicted arrangement of the inlet and outlet valves and lines of the beds permits the inlet and outlet of each bed to be closed, so that the function of each bed can be periodically shifted to function as that of one of the other two beds in the drawing.

In general, at least one sorbent bed is required, since even a single bed can function first in the position of bed 20, then in the position of bed 162, and finally in the position of bed 146, before functioning once again in the position of bed 20. More commonly, two or more beds are used. The removal of aromatic byproducts from the dehydrogenated product stream flowing in line 14 can range from a batchwise operation to an essentially continuous operation. Likewise, the off-stream sorbent bed purging and desorption functions may occur batchwise and relatively infrequently or essentially continuously.

A paraffin feed stream comprising an admixture of C10-C15 normal and branched paraffins is charged via line 10 and enters zone 12, where the dehydrogenation occurs. Olefins and aromatic byproducts are formed, and some diolefins may also be formed. A stream containing unreacted paraffins, monoolefins, and aromatic byproducts passes through line 14, valve 16, and line 18, and enters bed 20, which is on-stream for removal of aromatic byproducts. On-stream bed 20 contains a molecular sieve sorbent, which removes aromatic byproducts from the dehydrogenated product stream.

The effluent of on-stream bed 20 passes through line 24, valve 26, and line 28. It combines with a stream containing benzene, C10-C15 paraffins and olefins, and possibly a minor amount of n-pentane that is flowing in line 30. The combined stream flows through line 31, line 32, valve 36, and line 40. Since reactor 48 is on-stream, valve 92 is closed and no regenerant benzene is flowing through lines 88 and 96, so that the stream in line 40 is thus the inlet stream for on-stream reactor 48 flowing in line 44. The stream in line 44 may contain water but preferably the water content is minimized for most solid alkylation catalysts. Monoolefins alkylate benzene to produce alkylbenzenes in on-stream reactor 48. Reactor effluent containing alkylbenzenes, unreacted benzene, C10-C15 paraffins, n-pentane, and possibly water flows through line 52, line 56, valve 60, and line 64. Since reactor 50 is off-stream, valve 62 is closed so that there is no flow through line 58, valve 62, and line 66. Reactor effluent flows through line 68 to benzene column 70. Benzene column 70 produces a bottom stream in line 74 which contains alkylbenzenes and paraffins and which is sent to conventional product recovery facilities 80. Conventional product recovery facilities 80 separate the bottom stream into a paraffin-containing stream 76, a product stream containing the desired alkylbenzenes in line 78, and a stream containing heavier alkylbenzenes in line 82. Typically, conventional facilities 80 comprises one distillation column, called a paraffin column, which produces the paraffin-containing stream as an overhead stream, and a second distillation column, called an alkylbenzene column, which separates the paraffin column's bottom stream into an overhead stream containing the desired alkylbenzenes and a bottom stream containing the heavier alkylbenzenes.

A makeup benzene stream enters benzene column 70 in line 72. The overhead system of benzene column 70 may comprise a water boot or other conventional facilities for rejecting water from the process, since the makeup benzene stream may be wet. Benzene is recovered from the upper portion of benzene column 70. The drawing shows this benzene flowing in a single stream through line 84, with lines and valves that permit employing portions of this benzene for four different uses in the process. First, there is recycle benzene to the on-stream reactor (via line 85, valve 87, and line 89). Second, regenerant benzene is used for reactor 48 when reactor 48 is off-stream (via lines 86 and 88, valve 92, and line 96). Third, regenerant benzene is available for reactor 50 when reactor 50 is off-stream (via lines 86 and 90, valve 94, and line 98). Finally, benzene can be used for desorbing off-stream bed 146 (via line 100, valve 102, and line 104).

One portion of the overhead benzene stream flows to on-stream reactor 48 by flowing via lines 84 and 85, valve 87, and line 89. Valve 87 can be used to regulate the flow rate of this portion in order to maintain a desired quantity of benzene flowing to on-stream reactor 48. This portion combines with a stream containing benzene, C10-C15 paraffins and olefins, and possibly a minor amount of n-pentane that is flowing in line 120 to form the stream flowing in line 30. The stream in line 31 flows through line 32, valve 36, line 40, and line 44, and enters on-stream reactor 48.

When off-stream reactor 50 is undergoing regeneration using benzene as regenerant, another portion of the overhead stream in line 84 passes to reactor 50. This portion flows through line 84, line 86, line 90, valve 94, and line 98. Since reactor 50 is off-stream, valve 38 is closed and there is no flow through lines 34 and 42. Consequently, the portion flowing in line 98 flows through line 46 and enters reactor 50. This portion washes and/or reacts away heavy byproducts from the alkylation catalyst that cause the catalyst to deactivate. The effluent from off-stream reactor 50 contains benzene as well as these byproducts, which can include polynuclear hydrocarbons, polyalkylated aromatics, and olefin oligomers. While reactor 50 is off-stream, valve 130 is open, so that the effluent flows through line 54, line 126, valve 130, and line 134. There is no flow through lines 128 and 136 when reactor 48 is on-stream since valve 132 is closed then, and thus the stream in line 134 passes into line 106. Line 106 with its sample connection 108 thus carries effluent from either or any off-stream reactor undergoing regeneration. When the flow of effluent in line 106 exceeds the desired flow for passing to one and/or both of lines 110 and 114, then valve 63 can be opened and some of the effluent flowing in line 106 can flow to benzene column 70 through line 61, valve 63, and line 65. When valve 63 is closed, regeneration effluent does not flow into line 61 but instead flows through line 109 to the junction with lines 110 and 114.

Although either or both of valves 112 and 116 may be open when a reactor such as 50 is off-stream being regenerated, preferably at any time during the regeneration only one of valves 112 and 116 is open and the other is closed. More preferably and specifically for the previously described three-step regeneration method, when off-stream reactor 50 is undergoing the first regeneration heat-up step, the effluent of the off-stream reactor 50 can pass to the on-stream reactor 48. To accomplish this, valve 116 is opened and valve 112 is closed. Thus, the effluent of off-stream reactor 50 flows through line 54, line 126, valve 130, line 134, line 106, line 109, line 114, valve 116, and line 118. After flowing through line 118, the effluent combines with a stream flowing in line 172 which contains benzene, C10-C15 paraffins and olefins, and possibly a minor amount of n-pentane to form the stream flowing in line 120. The stream in line 120 combines with the stream in line 89 to form the stream in line 30. Thus, at least a portion of the benzene in the effluent of the off-stream reactor 50 undergoing the first step of regeneration passes to the on-stream reactor 48.

When the first step of regeneration is completed, as determined by analyses of the effluent at sample connection 108, the effluent of the off-stream reactor 50 can pass to the off-stream sorbent bed 146 undergoing desorption. To do this, valve 116 is closed and valve 112 is opened. During the second (hold) and third (cool-down) steps of the three-step regeneration method, the effluent of off-stream reactor 50, after discharging from line 109, flows through line 110, valve 112, and line 140. If the flow of regeneration effluent through line 140 is sufficient for desorption of off-stream sorbent bed 146, then there is no need to supplement the flow in line 140 with additional benzene from benzene column 70. In that case, valve 102 is closed, and there is no flow of benzene through line 100, valve 102, and line 104.
Accordingly, the regeneration effluent in line 140 flows through line 122, valve 142, and line 144, and into off-stream sorbent bed 146.

Usually, when an alkylation reactor, such as 50, is undergoing regeneration and its effluent is flowing to a sorbent bed such as 146 that is undergoing desorption, the effluent of the alkylation reactor undergoing regeneration will provide a sufficient flow rate of benzene to desorb the off-stream sorbent bed. If, however, no alkylation reactor is being regenerated, or if the rate of benzene flow from the alkylation reactor being regenerated to the sorbent bed undergoing desorption is insufficient to desorb the sorbent bed, then benzene must be supplied to the sorbent bed undergoing desorption from a source other than the effluent of any alkylation reactor undergoing regeneration. In these circumstances, benzene is made available by opening valve 102 so that benzene flows through line 100, valve 102, and line 104. from there, the benzene combines with the flow from line 140, if any, and flows to the off-steam bed 146 via lines 122 and 144.

In one embodiment of the present invention, the effluent of sorbent bed 146 undergoing desorption flows through line 148, valve 150, and line 152 to separation zone 154. The effluent of bed 162 which is undergoing off-stream sorbent bed purging flows through line 164, valve 166, and line 168, and also enters separation zone 154. Separation zone 154 separates the entering streams into a low-boiling stream in line 156 that contains the purge compound (e.g., n-pentane), a high-boiling stream in line 170 that contains aromatic byproducts desorbed from the sorbent bed 146, and an intermediate-boiling stream in line 172 that contains benzene and paraffins and olefins that have been purged from sorbent bed 162. The low-boiling stream in line 156 is recycled to sorbent bed 162, the high-boiling stream in line 170 is rejected from the process, and the intermediate boiling stream combines with the stream flowing in line 118, if any, so that the paraffins and olefins in line 172 ultimately pass to the on-stream reactor 48. Thus, at least a portion of the benzene in the effluent of the off-stream reactor 50 undergoing the second and/or third steps of-regeneration also passes to the on-stream reactor 48, albeit by way of the off-stream sorbent bed 146 and separation zone 154.

Separation zone 154 may be any suitable arrangement of distillation columns, such as two distillation columns in series. The stream flowing in line 152 passes to a first column, from which the stream flowing in line 170 is rejected as a bottom stream. The first column produces an overhead stream, which passes to a second column along with the stream flowing in line 168. The stream flowing through line 156 is recovered from the overhead of the second column, and the stream flowing in line 172 is recovered as a bottom stream from the second column.

Separation zone 154 can also reject from the process the gum-like materials including color bodies and/or olefinic compounds removed from the off-stream reactor 50, when the effluent of off-stream reactor 50 passes to off-stream sorbent bed 146. It has been observed that at typical desorption conditions the gum-like materials are not sorbed on the sorbent in off-stream sorbent bed 146. These relatively high-boiling compounds can pass to separation zone 154, where they can be rejected in the high-boiling stream in line 170 or in another high-boiling stream. Another method of rejecting these gum-like materials from the process, without passing the effluent of the off-stream reactor 50 to either the off-stream sorbent bed 146 or to the conventional product recovery facilities 80, is to pass the effluent of off-stream reactor 50 to separation zone 154. This is another more-direct way of rejecting these gum-like materials in a high-boiling stream from separation zone 154. Thus, this invention can help prevent these gum-like materials from passing to benzene column 70 and product recovery facilities 80.

By keeping these gum-like materials from entering benzene column 70 and product recovery facilities 80, this invention can improve the bromine index of the alkylated product. This invention can control the bromine index of the alkylated product at a target specification or within a specified range by regulating the flow of off-stream reactor 50 effluent to off-stream sorbent bed 146. Production of high-quality alkylated product, having a bromine index as measured by UOP Method 304-90 of less than 20, preferably of less than 10, can be controlled. This invention can also be used to control the production of alkylated product having a bromine index as measured by UOP Method 304-90 of greater than 20, of up to 100, or of even more than 100. Information on UOP Method 304-90, "Bromine Number and Bromine Index of Hydrocarbons by Potentiometric Titration," is available from ASTM International.

It is believed that the alkylated product of this invention has a Saybolt color of more than +25, preferably of +29 or more, and more preferably +30 or more. Also, it is well known that improving the bromine index of the alkylated product leads to an improvement in the Klett color index of sulfonated alkylbenzenes produced therefrom.

The functions of the two alkylation reactors 48 and 50 may be switched. When alkylation reactor 48 is off-stream and alkylation reactor 50 is on-stream, the effluent of reactor 48 may be passed to reactor 50 during certain periods of the regeneration of reactor 48, as previously described for the case of alkylation reactor 48 being on-stream and alkylation reactor 50 being off-stream. Thus, during regeneration of reactor 48, when valve 132 is open and valve 60 is closed, the effluent of reactor 48 may pass through line 128, valve 132, line 136, line 106, and line 109. With valve 112 closed and valve 116 open, the effluent flows through line 114, valve 116, and line 118. From line 118, the effluent combines with the stream flowing in line 172 and the combined stream in line 120, including benzene from the off-stream reactor 48 effluent, ultimately flows to reactor 50 via lines 30, 31, 34, 42, and 46. A sample of the effluent of reactor 48 may be taken for analysis at sample connection 108. When the regeneration effluent of reactor 48 is passed to off-stream sorbent bed 146, valve 112 is opened and valve 116 is closed, so that the effluent passes through line 110, valve 112, line 140, line 122, valve 142, and line 144 to sorbent bed 146. As described previously, the effluent of sorbent bed 146 flows to separation zone 154, and benzene recovered in line 172 enters line 120 and ultimately passes to on-stream reactor 50.

## Claims

1. A process for producing a alkyl aromatic, the process comprising:
a) producing a monoolefin and forming aromatic byproducts from an olefin-producing feed during the production of the monoolefin, and forming an olefinic stream comprising the monoolefin and aromatic byproducts;
b) removing aromatic byproducts from at least a portion of the olefin-producing feed or the olefinic stream in an on-stream aromatic byproducts removal bed containing a sorbent at sorption conditions effective to remove the aromatic byproducts;
c) passing a feed aromatic and at least a portion of the olefinic stream comprising the monoolefin to an on-stream alkylation reactor, alkylating the feed aromatic with the monoolefin in the presence of solid alkylation catalyst in the on-stream alkylation reactor at alkylation conditions to form a product aromatic, the alkylation conditions being sufficient to at least partially deactivate the solid alkylation catalyst in the on-stream alkylation reactor, and recovering from the on-stream alkylation reactor an on-stream reactor effluent stream comprising the product aromatic;
d) recovering the product aromatic from at least a portion of the on-stream reactor effluent stream;
e) contacting solid alkylation catalyst in an off-stream alkylation reactor with the feed aromatic, wherein the solid alkylation catalyst in the off-stream alkylation reactor is at least partially deactivated, wherein the off-stream alkylation reactor operates at regeneration conditions sufficient to at least partially reactivate the solid alkylation catalyst in the off-stream alkylation reactor, and recovering from the off-stream alkylation reactor an off-stream reactor effluent stream comprising the feed aromatic;
f) passing at least a portion of the off-stream reactor effluent stream to an off-stream aromatic byproducts removal bed containing sorbent, wherein the sorbent in the off-stream aromatic byproducts removal bed contains sorbed aromatic byproducts, to at least partially desorb the aromatic byproducts from the sorbent in the off-stream aromatic byproducts removal bed at desorption conditions, and recovering from the off-stream aromatic byproducts removal bed an off-stream bed effluent stream comprising the feed aromatic;
g) passing at least a portion of the feed aromatic in the off-stream bed effluent stream to the on-stream alkylation reactor;
h) at least intermittently shifting the functions of the on-stream alkylation reactor and the off-stream alkylation reactor by operating the on-stream alkylation reactor to function as the off-stream alkylation reactor and operating the off-stream alkylation reactor to function as the on-stream alkylation reactor; and
i) at least intermittently shifting the functions of the on-stream aromatic byproducts removal bed and the off-stream aromatic byproducts removal bed by operating the off-stream aromatic byproducts removal bed to function as the on-stream aromatic byproducts removal bed and operating the on-stream aromatic byproducts removal bed to function as the off-stream aromatic byproducts removal bed.

2. The process of Claim 1 wherein the monoolefin is a C₆-C₂₂ monoolefin, and the feed aromatic is benzene.

3. The process of Claim 1 further **characterized in that** the regeneration conditions comprise a regeneration temperature of from 200°C to the critical temperature of the feed aromatic.

4. The process of Claim 1 further **characterized in that** the on-stream bed effluent stream has a concentration of aromatic byproducts of less than 2 wt-%.

5. The process of Claim 1, wherein recovering the product aromatic from at least a portion of the on-stream reactor effluent stream comprises passing at least a portion of the on-stream reactor effluent stream to a product recovery section, and recovering from the product recovery section an alkylated product stream comprising the product aromatic and having a bromine index of less than 20.

6. The process of Claim 1 wherein the passing of at least a portion of the feed aromatic in the off-stream bed effluent stream to the on-stream alkylation reactor comprises passing at least a portion of the off-stream bed effluent stream to a separation zone, recovering a return stream comprising the feed aromatic from the separation zone, and passing at least a portion of the return stream to the on-stream alkylation reactor.

7. The process of Claim 1 further **characterized in that** the olefin-producing feed comprises paraffins and the olefin-producing feed is recovered from the at least a portion of the on-stream reactor effluent stream.

8. The process of claim 1, wherein the process for regenerating the solid catalyst comprises:
a) contacting a solid catalyst having deposits thereon in an off-stream alkylation reactor with a feed aromatic at a temperature between 120°C and 170°C recovering a regeneration effluent comprising the feed aromatic from the off-stream alkylation reactor, and passing at least a portion of the regeneration effluent comprising the feed aromatic to an on-stream alkylation reactor containing the solid catalyst; and
b) increasing the inlet temperature from 50°C to 200°C above the temperature of step (a) so that the regeneration effluent further comprises the deposits, passing at least a portion of the regeneration effluent comprising the feed aromatic and the deposits to a bed containing a sorbent having aromatic compounds sorbed thereon to desorb the aromatic compounds, recovering a desorption effluent from the bed wherein the desorption effluent comprises the feed aromatic, the aromatic compounds, and the deposits, separating at least a portion of the desorption effluent in a separation section into a light stream comprising the feed aromatic and a heavy stream comprising the deposits and the aromatic compounds, and passing at least a portion of the light stream comprising the feed aromatic to the on-stream alkylation reactor.

9. The process of claim 8 further comprising reducing the inlet regeneration temperature at the end of step (b) to the alkylation temperature.

## Patentansprüche

1. Verfahren zur Herstellung eines Alkylaromaten, umfassend:
a) Herstellung eines Monoolefins und Bildung von aromatischen Nebenprodukten aus einem olefinproduzierenden Einsatz während der Herstellung des Monoolefins und Bildung eines olefinischen Stroms, der das Monoolefin und aromatische Nebenprodukte umfaßt;
b) Entfernung von aromatischen Nebenprodukten aus mindestens einem Teil des olefinproduzierenden Einsatzes oder des olefinischen Stroms in einem sich im Betrieb befindenden Bett zur Entfernung von aromatischen Nebenprodukten, das ein Sorptionsmittel enthält, unter Sorptionsbedingungen, die zur Entfernung der aromatischen Nebenprodukte effektiv sind;
c) Zuführung eines Einsatz-Aromaten und mindestens eines Teils des das Monoolefin umfassenden olefinischen Stroms zu einem sich im Betrieb befindenden Alkylierungsreaktor, Alkylierung des Einsatz-Aromaten mit dem Monoolefin in dem sich im Betrieb befindenden Alkylierungsreaktor unter Alkylierungsbedingungen in Gegenwart von festem Alkylierungskatalysator zur Bildung eines Produkt-Aromaten, wobei die Alkylierungsbedingungen zur zumindest teilweisen Desaktivierung des festen Alkylierungskatalysators in dem sich im Betrieb befindenden Alkylierungsreaktor ausreichen, und Gewinnung eines Austragsstroms des sich im Betrieb befindenden Reaktors, der den Produkt-Aromaten umfaßt, aus dem sich im Betrieb befindenden Alkylierungsreaktor;
d) Gewinnung des Produkt-Aromaten aus mindestens einem Teil des Austragsstroms des sich im Betrieb befindenden Reaktors;
e) Inberührungbringen von festem Alkylierungskatalysator mit dem Einsatz-Aromaten in einem sich außer Betrieb befindenden Alkylierungsreaktor, wobei der feste Alkylierungskatalysator in dem sich außer Betrieb befindenden Alkylierungsreaktor zumindest teilweise desaktiviert ist, wobei der sich außer Betrieb befindende Alkylierungsreaktor bei Regenerationsbedingungen arbeitet, die zur zumindest teilweisen Reaktivierung des festen Alkylierungskatalysators in dem sich außer Betrieb befindenden Alkylierungsreaktor ausreichen, und Gewinnung eines Austragsstroms des sich außer Betrieb befindenden Reaktors, der den Einsatz-Aromaten enthält, aus dem sich außer Betrieb befindenden Alkylierungsreaktor;
f) Zuführung mindestens eines Teil des Austragsstroms des sich außer Betrieb befindenden Reaktors zu einem sich außer Betrieb befindenden Bett zur Entfernung von aromatischen Nebenprodukten, das Sorptionsmittel enthält, wobei das Sorptionsmittel in dem sich außer Betrieb befindenden Bett zur Entfernung von aromatischen Nebenprodukten sorbierte aromatische Nebenprodukte enthält, um die aromatischen Nebenprodukte unter Desorptionsbedingungen zumindest teilweise von dem Sorptionsmittel in dem sich außer Betrieb befindenden Bett zur Entfernung von aromatischen Nebenprodukten zu desorbieren, und Gewinnung eines Austragsstroms des sich außer Betrieb befindenden Betts, der den Einsatz-Aromaten umfaßt, aus dem sich außer Betrieb befindenden Bett zur Entfernung von aromatischen Nebenprodukten;
g) Zuführung mindestens eines Teils des Einsatz-Aromaten in dem Austragsstrom des sich außer Betrieb befindenden Betts zu dem sich im Betrieb befindenden Alkylierungsreaktor;
h) zumindest absatzweises Umstellen der Funktionen des sich im Betrieb befindenden Alkylierungsreaktors und des sich außer Betrieb befindenden Alkylierungsreaktors durch Betreiben des sich im Betrieb befindenden Alkylierungsreaktors als sich außer Betrieb befindender Alkylierungsreaktor und des sich außer Betrieb befindenden Alkylierungsreaktors als sich im Betrieb befindender Alkylierungsreaktor; und
i) zumindest absatzweises Umstellen der Funktionen des sich im Betrieb befindenden Betts zur Entfernung von aromatischen Nebenprodukten und des sich außer Betrieb befindenden Betts zur Entfernung von aromatischen Nebenprodukten durch Betreiben des sich außer Betrieb befindenden Betts zur Entfernung von aromatischen Nebenprodukten als sich im Betrieb befindendes Bett zur Entfernung von aromatischen Nebenprodukten und des sich im Betrieb befindenden Betts zur Entfernung von aromatischen Nebenprodukten als sich außer Betrieb befindendes Bett zur Entfernung von aromatischen Nebenprodukten.

2. Verfahren nach Anspruch 1, bei dem es sich bei dem Monoolefin um ein C₆-C₂₂-Monoolefin handelt und es sich bei dem Einsatz-Aromaten um Benzol handelt.

3. Verfahren nach Anspruch 1, ferner **dadurch gekennzeichnet, daß** die Regenerationsbedingungen eine Regenerationstemperatur von 200°C bis zur kritischen Temperatur des Einsatz-Aromaten umfassen.

4. Verfahren nach Anspruch 1, ferner **dadurch gekennzeichnet, daß** der Austragsstrom des sich im Betrieb befindenden Betts eine Konzentration an aromatischen Nebenprodukten von weniger als 2 Gew.-% aufweist.

5. Verfahren nach Anspruch 1, bei dem die Gewinnung des Produkt-Aromaten aus mindestens einem Teil des Austragsstroms des sich im Betrieb befindenden Reaktors die Zuführung mindestens eines Teils des Austragsstroms des sich im Betrieb befindenden Reaktors zu einem Produktgewinnungsabschnitt und die Gewinnung eines Stroms von alkyliertem Produkt, der den Produkt-Aromaten umfaßt und eine Bromzahl von weniger als 20 aufweist, aus dem Produktgewinnungsabschnitt umfaßt.

6. Verfahren nach Anspruch 1, bei dem die Zuführung mindestens eines Teils des Einsatz-Aromaten in dem Austragsstrom des sich außer Betrieb befindenden Betts zu dem sich im Betrieb befindenden Alkylierungsreaktor die Zuführung mindestens eines Teils des Austragsstroms des sich außer Betrieb befindenden Betts zu einer Trennzone, die Gewinnung eines Rückführungsstroms, der den Einsatz-Aromaten umfaßt, aus der Trennzone und die Zuführung mindestens eines Teils des Rückführungsstroms zu dem sich im Betrieb befindenden Alkylierungsreaktor umfaßt.

7. Verfahren nach Anspruch 1, ferner **dadurch gekennzeichnet, daß** der olefinproduzierende Einsatz Paraffine umfaßt und der olefinproduzierende Einsatz aus dem mindestens einem Teil des Austragsstroms des sich im Betrieb befindenden Reaktors gewonnen wird.

8. Verfahren nach Anspruch 1, bei dem das Verfahren zur Regeneration des festen Katalysators folgendes umfaßt:
a) Inberührungbringen eines festen Katalysators mit Ablagerungen darauf in einem sich außer Betrieb befindenden Alkylierungsreaktor mit einem Einsatz-Aromaten bei einer Temperatur zwischen 120°C und 170°C unter Gewinnung eines Regenerationsaustragsstroms, der den Einsatz-Aromaten aus dem sich außer Betrieb befindenden Alkylierungsreaktor umfaßt, und Zuführung mindestens eines Teils des Regenerationsaustragsstroms, der den Einsatz-Aromaten umfaßt, zu einem sich im Betrieb befindenden Alkylierungsreaktor, der den festen Katalysator enthält; und
b) Erhöhung der Einlaßtemperatur von 50°C bis 200°C über der Temperatur von Schritt (a), so daß der Regenerationsaustragsstrom ferner die Ablagerungen umfaßt, Zuführung mindestens eines Teils des Regenerationsaustragsstroms, der den Einsatz-Aromaten und die Ablagerungen umfaßt, zu einem Bett, das ein Sorptionsmittel mit darauf absorbierten aromatischen Verbindungen enthält, um die aromatischen Verbindungen zu desorbieren, Gewinnung eines Desorptionsaustragsstroms aus dem Bett, wobei der Desorptionsaustragsstrom den Einsatz-Aromaten, die aromatischen Verbindungen und die Ablagerungen umfaßt, Trennung mindestens eines Teils des Desorptionsaustragsstroms in einem Trennabschnitt in einen leichten Strom, der den Einsatz-Aromaten umfaßt, und einen schweren Strom, der die Ablagerungen und die aromatischen Verbindungen umfaßt, und Zuführung mindestens eines Teils des leichten Stroms, der den Einsatz-Aromaten umfaßt, zu dem sich im Betrieb befindenden Alkylierungsreaktor.

9. Verfahren nach Anspruch 8, ferner umfassend die Verringerung der Einlaßregenerationstemperatur am Ende von Schritt (b) auf die Alkylierungstemperatur.

## Revendications

1. Procédé de production d'un alkylaromatique, le procédé comprenant les étapes consistant à :
a) produire une monooléfine et former des sous-produits aromatiques à partir d'une charge d'alimentation pour la production d'oléfines pendant la production de la monooléfine, et former un courant oléfinique comprenant la monooléfine et les sous-produits aromatiques ;
b) éliminer les sous-produits aromatiques d'au moins une partie de la charge d'alimentation pour la production d'oléfines ou du courant oléfinique dans un lit d'élimination des sous-produits aromatiques en ligne contenant un sorbant dans des conditions de sorption efficaces pour éliminer les sous-produits aromatiques ;
c) envoyer une charge d'alimentation d'aromatique et au moins une partie du courant oléfinique comprenant la monooléfine dans un réacteur d'alkylation en ligne, alkyler la charge d'alimentation d'aromatique avec la monooléfine en présence d'un catalyseur d'alkylation solide dans le réacteur d'alkylation en ligne dans des conditions d'alkylation pour former un produit aromatique, les conditions d'alkylation étant suffisantes pour désactiver au moins partiellement le catalyseur d'alkylation solide dans le réacteur d'alkylation en ligne, et récupérer dans le réacteur d'alkylation en ligne un courant d'effluent du réacteur en ligne comprenant le produit aromatique ;
d) récupérer le produit aromatique à partir d'au moins une partie du courant d'effluent du réacteur en ligne ;
e) mettre en contact le catalyseur d'alkylation solide, dans un réacteur d'alkylation hors ligne avec la charge d'alimentation d'aromatique, le catalyseur d'alkylation solide dans le réacteur d'alkylation hors ligne étant au moins partiellement désactivé, le réacteur d'alkylation hors ligne fonctionnant dans des conditions de régénération suffisantes pour réactiver au moins partiellement le catalyseur d'alkylation solide dans le réacteur d'alkylation hors ligne, et récupérer à partir du réacteur d'alkylation hors ligne un courant d'effluent du réacteur hors ligne comprenant la charge d'alimentation d'aromatique ;
f) envoyer au moins une partie du courant d'effluent du réacteur hors ligne dans un lit d'élimination des sous-produits aromatiques hors ligne contenant un sorbant, dans lequel le sorbant dans le lit d'élimination des sous-produits aromatiques hors ligne contient des sous-produits aromatiques sorbés, pour désorber au moins partiellement les sous-produits aromatiques du sorbant dans le lit d'élimination des sous-produits aromatiques hors ligne dans des conditions de désorption, et récupérer à partir des sous-produits aromatiques hors ligne un courant d'effluent du lit hors ligne comprenant la charge d'alimentation d'aromatique;
g) envoyer au moins une partie de la charge d'alimentation d'aromatique se trouvant dans le courant d'effluent du lit hors ligne dans le réacteur d'alkylation en ligne;
h) au moins par intermittence, changer les fonctions du réacteur d'alkylation en ligne et du réacteur d'alkylation hors ligne en faisant fonctionner le réacteur d'alkylation en ligne comme le réacteur d'alkylation hors ligne et en faisant fonctionner le réacteur d'alkylation hors ligne comme le réacteur d'alkylation en ligne ; et
i) au moins par intermittence, changer les fonctions du lit d'élimination des sous-produits aromatiques en ligne et du lit d'élimination des sous-produits aromatiques hors ligne en faisant fonctionner le lit d'élimination des sous-produits aromatiques hors ligne comme le lit d'élimination des sous-produits aromatiques en ligne et en faisant fonctionner le lit d'élimination des sous-produits aromatiques en ligne comme le lit d'élimination des sous-produits aromatiques hors ligne.

2. Procédé selon la revendication 1 dans lequel la monooléfine est une monooléfine en C₆-C₂₂ et la charge d'alimentation d'aromatique est le benzène.

3. Procédé selon la revendication 1 **caractérisé en outre par le fait que** les conditions de régénération comprennent une température de régénération allant de 200°C à la température critique de la charge d'alimentation d'aromatique.

4. Procédé selon la revendication 1 **caractérisé en outre par le fait que** le courant d'effluent du lit en ligne a une concentration en sous-produits aromatiques inférieure à 2% en poids.

5. Procédé selon la revendication 1 dans lequel la récupération du produit aromatique à partir d'au moins une partie du courant d'effluent du réacteur en ligne comprend le fait d'envoyer au moins une partie du courant d'effluent du réacteur en ligne dans une section de récupération du produit, et de récupérer à partir de la section de récupération du produit un courant de produit alkylé comprenant le produit aromatique et ayant un indice de brome inférieur à 20.

6. Procédé selon la revendication 1 dans lequel le fait d'envoyer au moins une partie de la charge d'alimentation d'aromatique se trouvant dans le courant d'effluent du lit hors ligne dans le réacteur d'alkylation en ligne comprend le fait d'envoyer au moins une partie du courant d'effluent du lit hors ligne dans une zone de séparation, de récupérer un courant de retour comprenant la charge d'alimentation d'aromatique à partir de la zone de séparation, et d'envoyer au moins une partie du courant de retour dans le réacteur d'alkylation en ligne.

7. Procédé selon la revendication 1 **caractérisé en outre par le fait que** la charge d'alimentation pour la production d'oléfines comprend des paraffines et la charge d'alimentation pour la production d'oléfines est récupérée à partir d'au moins une partie du courant d'effluent du réacteur en ligne.

8. Procédé selon la revendication 1 dans lequel le procédé de régénération du catalyseur solide comprend les étapes consistant à :
a) mettre en contact un catalyseur solide sur lequel se trouvent des dépôts dans un réacteur d'alkylation hors ligne avec une charge d'alimentation d'aromatique à une température comprise entre 120°C et 170°C, récupérer un effluent de régénération comprenant la charge d'alimentation d'aromatique à partir du réacteur d'alkylation hors ligne, et envoyer au moins une partie de l'effluent de régénération comprenant la charge d'alimentation d'aromatique dans un réacteur d'alkylation en ligne contenant le catalyseur solide ; et
b) élever la température d'entrée de 50°C à 200°C au-dessus de la température de l'étape (a) de façon à ce que l'effluent de régénération comprenne en outre les dépôts, envoyer au moins une partie de l'effluent de régénération comprenant la charge d'alimentation d'aromatique et les dépôts dans un lit contenant un sorbant sur lequel sont sorbés des composés aromatiques afin de désorber les composés aromatiques, récupérer un effluent de désorption à partir du lit, l'effluent de désorption comprenant la charge d'alimentation d'aromatique, les composés aromatiques et les dépôts, séparer au moins une partie de l'effluent de désorption dans une section de séparation pour former un courant léger comprenant la charge d'alimentation d'aromatique et un courant lourd comprenant les dépôts et les composés aromatiques, et envoyer au moins une partie du courant léger comprenant la charge d'alimentation d'aromatique dans le réacteur d'alkylation en ligne.

9. Procédé selon la revendication 8 comprenant en outre le fait d'abaisser la température de régénération à l'entrée, à la fin de l'étape (b), jusqu'à la température d'alkylation.
